(19) Europäisches Patentamt / European Patent Office / Office européen des brevets

(11) **EP 3 006 069 A1**

(12) **EUROPEAN PATENT APPLICATION**

(43) Date of publication:
**13.04.2016  Bulletin 2016/15**

(51) Int Cl.:
**A61M 5/315** (2006.01)    **B29C 33/42** (2006.01)
**B29C 43/18** (2006.01)

(21) Application number: **15188759.3**

(22) Date of filing: **07.10.2015**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA**

(30) Priority: **10.10.2014  JP 2014209223**

(71) Applicant: **Sumitomo Rubber Industries, Ltd.
Hyogo-ken (JP)**

(72) Inventors:
• ISHIDA, Naoyuki
  Kobe-shi, Hyogo 651-0072 (JP)
• NAKANO, Hiroaki
  Kobe-shi, Hyogo 651-0072 (JP)
• MATSUTANI, Yuichiro
  Kobe-shi, Hyogo 651-0072 (JP)
• KANEKO, Hiroyuki
  Kobe-shi, Hyogo 651-0072 (JP)

(74) Representative: **Manitz, Finsterwald & Partner
GbR
Martin-Greif-Strasse 1
80336 München (DE)**

(54) **GASKET FOR PREFILLED SYRINGE**

(57)    A gasket (10) for a prefilled syringe is provided, which has a generally cylindrical outer shape, and includes a liquid contact portion (13) provided on one of opposite end faces, a thread hole (15) provided in the other end face for attachment of a plunger, and at least two annular projections (17A,17B) provided on a peripheral surface (16) and spaced from each other by an annular trough (18) in a gasket height direction (gasket axial direction) extending from the one end face to the other end face, wherein a diameter difference (A) (= a1 - a2) between a diameter (a1) of the annular projections (17A, 17B) and a diameter (a2) of the annular trough and a diameter difference (B) (= b1 - b2) between a thread root diameter (b1) and a thread ridge diameter (b2) of an internal thread portion of the thread hole satisfy a ratio relationship of A : B = 1 : (2 to 4).

FIG. 1

**Description**

TECHNICAL FIELD

The present invention relates to a gasket for a prefilled syringe.

BACKGROUND ART

In recent years, syringes including a syringe barrel prefilled with a liquid drug have been increasingly used because of their handling ease and capability of preventing medical accidents such as misadministration of liquid drugs. In such a prefilled syringe, a gasket is provided in the syringe barrel, and a distal portion of the syringe barrel to which an injection needle is attached is closed with a nozzle cap. The liquid drug is sealed in an inside space of the syringe barrel defined between the gasket and the distal portion. When the liquid drug is to be administered, the nozzle cap is removed from the distal portion, and then the injection needle is attached to the distal portion. Further, a plunger is connected to the gasket, and pushed toward the distal portion to slide the gasket. Thus, the liquid drug is administered.
The gasket for the syringe is required to have gas tightness and low-friction slidability.
Where a gasket laminated with an inert film is used, in general, a user can properly move the gasket by pushing the plunger by one hand in using the prefilled syringe even in the absence of silicone on a surface of the gasket. However, the inert film is not comparable in shape followability to rubber and, therefore, the gasket is required to have higher gas tightness when being inserted into the syringe barrel.
The gasket laminated with the inert film is produced by a so-called compression production method or a so-called injection production method employing a mold as typically described in JP2013-49236A. In the production method employing the mold, it is important to properly demold (remove) a product (gasket) from the mold. More specifically, when the product is demolded from the mold, it is necessary to leave the product in either one of a female die and a male die of the mold (specifically, in the male die). If this is impossible, the product is insufficiently demolded from the female die and still remains in the female die. This makes it difficult to control the dimensions of a peak portion of a peripheral surface of the gasket.
A typical example of the liquid to be contained in the prefilled syringe is a liquid drug to be injected in a human body. Therefore, the gasket to be used for the prefilled syringe is required to be highly clean. Accordingly, special care should be taken to prevent a thread portion provided in the gasket from being cracked or contaminated with rubber dust when the product (gasket) is demolded from the mold.

CITATION LIST

PATENT DOCUMENT

Patent Document 1: JP2013-49236A

SUMMARY OF THE INVENTION

PROBLEM TO BE SOLVED BY THE INVENTION

In view of the foregoing, it is an object of the present invention to provide a gasket imparted with excellent gas tightness and low-friction slidability by leaving the gasket in one of two dies of a mold when the gasket is demolded from the mold for production of the gasket.
It is another object of the present invention to provide a gasket which satisfies a high cleanliness requirement.

SOLUTION TO PROBLEM

According to the present invention, there is provided a gasket for a prefilled syringe, the gasket having a generally cylindrical outer shape, and including a liquid contact portion provided on one of opposite end faces, a thread hole provided in the other end face for attachment of a plunger, and at least two annular projections provided on a peripheral surface and spaced from each other by an annular trough in a gasket height direction (gasket axial direction) extending from the one end face to the other end face, wherein a diameter difference A (= a1 - a2) between the diameter a1 of the annular projections and the diameter a2 of the annular trough and a diameter difference B (= b1 - b2) between a thread root diameter b1 and a thread ridge diameter b2 of an internal thread portion of the thread hole satisfy a ratio relationship of A : B = 1 : (2 to 4).
In the present invention, at least the liquid contact portion and the annular projections of the gasket may be covered with an inert film.

In the present invention, the inert film is preferably made of a material comprising at least one of PTEF, ETEF, PFA, FEP, PCTFE, PVDF and PVF, modified fluororesins obtained by modifying these resins, nylons and ultrahigh molecular weight PE.

In the present invention, the annular projections provided on the peripheral surface have a total width that is 20 to 70 % of the height of the gasket (as measured axially of the gasket).

In the present invention, the internal thread portion of the thread hole may have 1 to 3.5 thread turns.

EFFECTS OF THE INVENTION

In the inventive gasket for the prefilled syringe, the diameter difference A between the peak diameter and the trough diameter of the gasket periphery and the diameter difference B between the thread ridge diameter and the thread root diameter of the gasket thread portion is A : B = 1 : (2 to 4). Further, the gasket thread portion has 1 to 3.5 thread turns. In production of the gasket, therefore, the gasket can be smoothly demolded from a mold without a dimensional error of the peak portion. Since the total width (total length) of the annular projections is 20 to 70 % of the height of the overall gasket, the gasket has sufficient gas tightness even with the liquid contact portion and the sliding portion laminated with the inert film. Thus, the gasket prevents intrusion of foreign matter from the outside and leakage of a content liquid to the outside.

BRIEF DESCRIPTION OF THE DRAWINGS

FIG. 1 is a vertical sectional view schematically showing the construction of a gasket for a prefilled syringe according to one embodiment of the present invention.
FIGS. 2A and 2B are schematic sectional views for explaining a gasket production process.

EMBODIMENTS OF THE INVENTION

With reference to the attached drawings, one embodiment of the present invention will hereinafter be describe specifically.

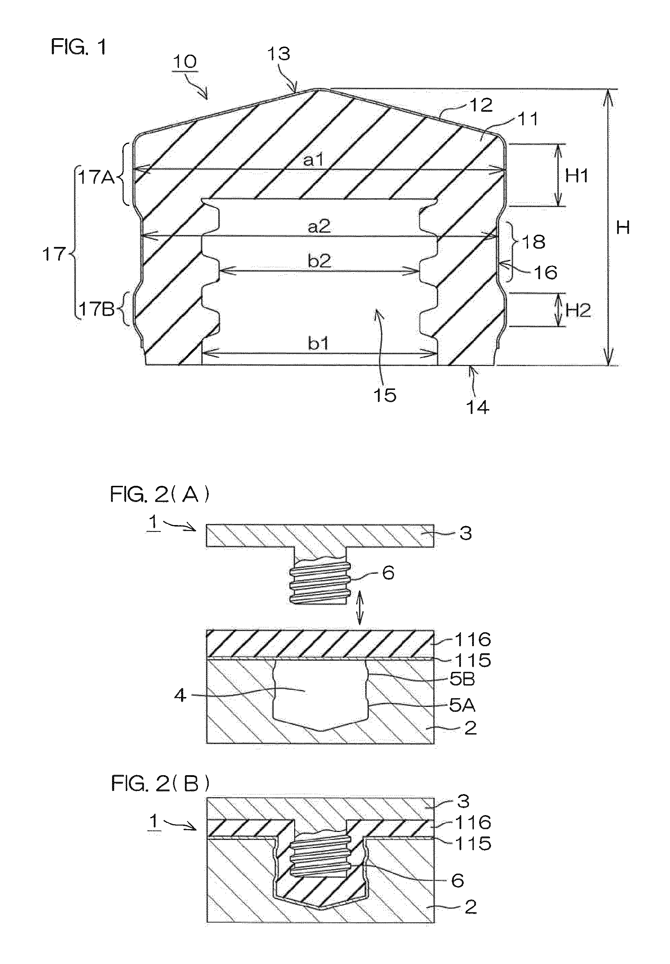

FIG. 1 is a vertical sectional view schematically showing the construction of a gasket 10 for a prefilled syringe according to one embodiment of the present invention.

The gasket 10 includes a main body 11 made of a rubber material, and an inert film layer 12 provided on a surface of the main body 11 to cover a part of the main body 11.

The main body 11 of the gasket 10 has a generally cylindrical outer shape. One end face 13 (upper end face in FIG. 1) of the main body 11 serves as a liquid contact surface 13 having a conical shape. The liquid contact surface 13 is covered with the inert film layer 12. The main body 11 of the gasket 10 has a thread hole 15 formed in the other end face (lower end face in FIG. 1) thereof for attachment of a plunger (not shown).

A peripheral surface 16 of the main body 11 of the gasket 10 is covered with the inert film layer 12. At least two annular projections 17A, 17B are provided on the peripheral surface 16 and spaced from each other by an annular trough 18. More specifically, the annular projection 17A is connected to a periphery of the liquid contact portion 13, and has an annular belt-like portion having a width H1 as measured in a height direction extending from the one end face 13 to the other end face 14 of the main body 11 (axially of the main body 11). The annular trough 18 is provided below the annular projection 17A (on a lower side as seen axially of the main body 11), and has a predetermined width (length) as measured axially of the main body 11. The annular projection 17B is provided below the annular trough 18 (on a lower side as seen axially of the main body 11), and has an annular belt-like portion having a width (length) H2 as measured axially of the main body 11.

Provided that the main body 11 has a height (axial length) H, a length ratio satisfies:

$$H : (H1 + H2) = 100 : (20\ to\ 70)$$

The annular projections 17A, 17B each have a diameter a1, and the annular trough 18 has a diameter a2. A diameter difference A between the annular projections 17A, 17B and the annular trough 18 is A = a1 - a2.

Further, the thread hole 15 has a thread portion formed in an inner peripheral surface thereof. The thread portion has 1 to 4 thread turns (3 thread turns in FIG. 1). The thread portion has a thread root diameter b1 and a thread ridge diameter b2, and a diameter difference B is B = b1 - b2.

The gasket 10 according to this embodiment is designed to have a configuration that satisfies a ratio relationship of A : B = 1 : (2 to 4).

The gasket 10 is configured in the aforementioned manner for the following reason:

A peak portion 17 (including the annular projections 17A, 17B) provided on the peripheral surface 16 of the gasket 10 is brought into intimate contact with an inner peripheral surface of a syringe barrel when the gasket is inserted into the syringe barrel. Therefore, the peak portion 17 is an important portion that ensures the gas tightness.

On the other hand, the trough 18 (annular trough) provided on the peripheral surface 16 of the gasket 10 is presupposed to be kept out of contact with the inner peripheral surface of the syringe barrel for suppression of unwanted increase in sliding resistance when the gasket is inserted into the syringe barrel.

Incidentally, the inner diameter of the syringe barrel is generally smaller than the outer diameter of the gasket 10. Therefore, the diameter a2 of the trough 18 should be sufficiently smaller than the diameter a1 of the peak portion 17. If not so, the trough 18 is brought into contact with the inner peripheral surface of the syringe barrel to increase the sliding resistance when the gasket is inserted into the syringe barrel.

When the gasket 10 is produced by molding with the use of a mold including a thread forming die (male die) and a peak forming die (female die), on the other hand, it is desirable to leave a gasket product on the thread forming die in order to properly demold the gasket product from the mold. If the product is to be left in the peak forming die (female die), the product will be insufficiently demolded from the female die and still remains in the female die. Therefore, the peak portion 17 is liable to have a smaller diameter a1. If the diameter a1 of the peak portion 17 cannot be properly controlled, it will be impossible to bring the peak portion 17 into intimate contact with the inner peripheral surface of the syringe barrel when the gasket 10 is inserted into the syringe barrel. This will reduce the gas tightness, resulting in intrusion of foreign matter from the outside and/or leakage of the sealed liquid drug to the outside.

In order to leave the gasket product on the thread forming die when the gasket product is demolded from the mold in the production of the gasket 10, the ratio of the diameter difference A between the peak portion 17 and the trough 18 of the gasket peripheral surface 16 to the diameter difference B between the thread root diameter b1 and the thread ridge diameter b2 of the gasket thread hole 15 desirably satisfies A : B = 1 : (3.0 to 4.0).

For the proper demolding of the product under the above conditions, the gasket thread portion desirably has 1 to 4 turns, more preferably 1 to 3.5 turns. The thread portion may have a single thread configuration or a double thread configuration. If the number of the thread turns is smaller, the product is liable to remain in the peak forming die because the product is retained on the thread forming die with an insufficient retention force in the demolding of the product. If the number of the thread turns is greater, the thread potion is more liable to be cracked or contaminated with rubber dust in the demolding of the product. The cracking is liable to reduce the cleanliness of the gasket, so that the gasket will be rejected. On the other hand, the rubber dust is liable to increase a process load with the need for removal thereof.

With the provision of the gasket 10 laminated with the inert film layer 12, the user of the prefilled syringe can properly use the prefilled syringe by one hand even in the absence of silicone on the inner peripheral surface of the syringe barrel. However, the inert film layer 12 is not comparable in shape followability to rubber (the main body 11 of the gasket 10). In order to increase the gas tightness of the gasket 10, therefore, the width (length) of the peak portion 17 is desirably 20 to 70 % of the height H of the overall gasket 10.

The rubber material to be used for the main body 11 of the gasket 10 desirably has an elastic modulus of not greater than 1.5 MPa, more preferably 0.8 to 1.2 MPa. Usable examples of the rubber material include IIR, IR, BR, SBR, EPDM, fluororubbers, silicone rubbers and thermoplastic elastomers.

Next, the mold to be used for the production of the gasket 10 and a production process for the gasket 10 will be briefly described. Examples of the mold to be used for the production of the gasket 10 include those disclosed in JP2013-49236A and JP2014-14698A.

FIGS. 2A and 2B are schematic sectional views for explaining a gasket production process employing a mold 1 for molding the gasket 10 for the prefilled syringe according to this embodiment.

The mold 1 includes a female die (lower die) 2 and a male die (upper die) 3. In FIGS. 2A and 2B, the male die 3 is vertically movable with respect to the female die 2. A heater (not shown) is connected to the female die 2 and the male die 3 for heating the female die 2 and the male die 3. Exemplary heat sources for the heater include an electric heater, steam and oil.

A material for the mold 1 including the female die 2 and the male die 3 is not particularly limited, but a known mold material may be used. Preferred examples of the material for the mold 1 include carbon steel and precipitation type stainless steel. The mold 1 may be produced by a cutting process, for example, by cutting a blank material by means of a cemented carbide tool, a coated cemented carbide tool, a cBN sintered tool or the like, and then grinding and mirror-polishing the resulting product.

The female die 2 has a cavity 4 recessed inward. The cavity 4 is configured so as to correspond to the outer shape of the gasket 10.

The cavity 4 of the female die 2 has annular projection forming portions 5A, 5B which respectively correspond to the annular projections 17A, 17B of the gasket 10. Mold surface portions (sliding surface forming portions) of the annular projection forming portions 5A, 5B which form the sealing annular belt-like portions (sliding surfaces) of the annular projections are mirror-finished so as to have an arithmetic average roughness Ra of not greater than 0.03 $\mu$m as measured with a cutoff value of 0.08 mm.

The male die 3 includes a projection 6 provided on a lower side thereof for forming the thread hole 15 of the gasket 10. The projection 6 is formed with a 3-turn thread for the attachment of the plunger.

In a molding step, the mold 1 is preheated before the molding of the gasket. The preheating temperature is preferably about 155°C to about 200°C.

Then, an inert film 115 and a kneaded material sheet 116 (unvulcanized rubber sheet) for the main body 11 of the gasket 10 are placed in superposition on an upper surface of the female die 2. Alternatively, the male die 3 (core) may be located on a lower side, and the female die 2 (cavity) may be located on an upper side. In this case, the unvulcanized rubber sheet on which the lamination film is superposed may be placed on the male die 3 (core).

The thickness of the inert film 115 may be properly adjusted according to the shape and the size of the gasket, but is preferably 50 to 200 $\mu$m.

The kneaded material sheet 116 is made of an elastic material, and forms the main body 11 (core of the gasket 10). The kneaded material sheet 116 is an unvulcanized rubber sheet produced by blending ingredients in a predetermined blend ratio and kneading the resulting mixture by means of an enclosed kneading machine or an open roll kneading machine, and forming the kneaded mixture into a sheet by mean of a calender or a sheet forming machine.

Then, the inert film 115 and the unvulcanized rubber sheet 116 having a predetermined weight and size are placed in superposition in the mold, and press-molded by means of a vacuum press. Molding conditions are not particularly limited, but may be properly determined. The molding temperature is preferably 155°C to 200°C, more preferably 165°C to 180°C, and the molding period is preferably 1 to 20 minutes, more preferably 3 to 15 minutes, further more preferably 5 to 10 minutes.

Thereafter, an unnecessary portion is cut away and removed from the molded gasket product, which is in turn cleaned, sterilized, dried and visually checked. Thus, the gasket 10 is completed.

Examples

Several products (sample gaskets) were produced. A production method and an evaluation method for the products will be described below.

The method described in JP2013-49236A was employed for the production of the products.

A mold used for the production of the products was made of stainless steel, and the surface roughness of the mold was adjusted to Ra = 0.3 to 0.5 with the use of alumina.

The products (sample gaskets) were each adapted for a 1-mL syringe, and each had a diameter of 3.5 mm.

The sliding resistance of each of the sample gaskets was measured with a measurement stroke of 20 mm at a measurement rate of 100/mm/min by means of a desk-top autograph (available from Shimadzu Corporation). A material for a syringe barrel was COP, and the syringe barrel had an inner diameter of 6.3 mm. If a gasket of a syringe has a sliding resistance of not greater than 9.0 N, a user can properly use the syringe by one hand. A sliding resistance of not greater than 7.0 N is more preferred. Therefore, a sample gasket having a sliding resistance of not greater than 7.0 N was rated as excellent (○), and a sample gasket having a sliding resistance of 0 to 9.0 N was rated as acceptable (△). A sample gasket having a sliding resistance of greater than 9.0 N was rated as unacceptable (×).

Further, the sample gaskets were each evaluated for gas tightness. The sample gaskets were each attached to a syringe barrel, and 1 mL of distilled water was injected into the syringe from a nozzle. Subsequently, the syringe was capped, and warmed in a warm bath at 70°C for one hour. Then, the syringe was visually checked for evaluation. A sample gasket which was free from liquid leakage after a lapse of 24 hours was rated as excellent (○), and a sample gasket which was free from liquid leakage immediately thereafter but suffered from the liquid leakage after a lapse of 24 hours was rated as acceptable (△). A sample gasket which suffered from the liquid leakage immediately thereafter was rated as unacceptable (×).

The evaluation results are shown below in Table 1.

Table 1

| | Comparative Example 1 | Comparative Example 2 | Example 1 | Example 2 | Example 3 | Example 4 | Example 5 | Example 6 | Comparative Example 3 |
|---|---|---|---|---|---|---|---|---|---|
| A (mm) | 0.75 | 0.75 | 0.5 | 0.5 | 0.5 | 0.5 | 0.5 | 0.5 | 0.5 |
| B (mm) | 1.5 | 1.5 | 1.5 | 1.5 | 1.5 | 1.5 | 1.5 | 2.0 | 3.0 |
| A : B | 1:2.0 | 1:2.0 | 1:3.0 | 1:3.0 | 1:3.0 | 1:3.0 | 1:3.0 | 1:4.0 | 1:6.0 |
| Number of thread turns | 2 | 2 | 2 | 2 | 2 | 1 | 4 | 2 | 2 |
| Percentage of length of peak portion to overall height | 70% | 40% | 40% | 70% | 20% | 40% | 40% | 40% | 40% |
| Gas tightness | × | × | ○ | ○ | △ | △ | ○ | ○ | ○ |
| Sliding resistance | △ | ○ | ○ | △ | ○ | ○ | ○ | ○ | ○ |
| Comprehensive evaluation | × | △ | ◎ | ○ | ○ | ○ | ○*1 | ◎ | △*2 |

*1: Thread portion was contaminated with rubber dust.
*2: Thread portion was cracked.

6

This application corresponds to Japanese Patent Application No. 2014-209223 filed in the Japan Patent Office on October 10, 2014, the disclosure of which is incorporated herein by reference in its entirety.

**Claims**

1.  A gasket for a prefilled syringe, the gasket comprising:

    a generally cylindrical main body having a liquid contact portion provided on one of opposite end faces thereof, and a thread hole provided in the other end face thereof for attachment of a plunger; and
    at least two annular projections provided on a peripheral surface of the main body and spaced from each other by an annular trough in a gasket height direction (gasket axial direction) extending from the one end face to the other end face;
    wherein a diameter difference A (= a1 - a2) between a diameter a1 of the annular projections and a diameter a2 of the annular trough and a diameter difference B (= b1 - b2) between a thread root diameter b1 and a thread ridge diameter b2 of an internal thread portion of the thread hole satisfy a ratio relationship of A : B = 1 : (2 to 4).

2.  The gasket according to claim 1, wherein at least the liquid contact portion and the annular projections are covered with an inert film.

3.  The gasket according to claim 2, wherein the inert film is made of a material comprising at least one of PTEF, ETEF, PFA, FEP, PCTFE, PVDF and PVF, modified fluororesins obtained by modifying these resins, nylons and ultrahigh molecular weight PE.

4.  The gasket according to any one of claims 1 to 3, wherein the annular projections provided on the peripheral surface have a total width that is 20 to 70 % of a height of the gasket as measured axially of the gasket.

5.  The gasket according to any one of claims 1 to 4, wherein the internal thread portion of the thread hole has 1 to 3.5 thread turns.

FIG. 1

FIG. 2( A )

FIG. 2( B )

Europäisches
Patentamt

European
Patent Office

Office européen
des brevets

# EUROPEAN SEARCH REPORT

Application Number

EP 15 18 8759

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2009/128265 A1 (AJINOMOTO KK [JP]; MURAMATSU YASUHIRO [JP]) 22 October 2009 (2009-10-22) | 1,4,5 | INV. A61M5/315 |
| Y | * figure 4 * | 3 | ADD. B29C33/42 |
| X | EP 2 781 231 A2 (SUMITOMO RUBBER IND [JP]) 24 September 2014 (2014-09-24) * paragraph [0020]; figures 1,2 * | 1,3-5 | B29C43/18 |
| X | EP 2 703 025 A1 (SUMITOMO RUBBER IND [JP]) 5 March 2014 (2014-03-05) * paragraph [0033]; figure 1 * | 1-4 | |
| X | EP 2 703 024 A1 (TAISEI KAKO CO [JP]) 5 March 2014 (2014-03-05) * figure 1 * | 1,4,5 | |
| X | WO 2011/125133 A1 (COKI ENGINEERING INC [JP]; YOTSUTSUJI AKIRA [JP]) 13 October 2011 (2011-10-13) * paragraphs [0047] - [0049]; figure 3 * | 1,3-5 | TECHNICAL FIELDS SEARCHED (IPC) |
| X | JP 2012 147859 A (SUMITOMO RUBBER IND) 9 August 2012 (2012-08-09) * paragraph [0024]; figures 2,4,5 * | 1,3-5 | A61M B29C |
| X | WO 2004/044464 A1 (TERUMO CORP [JP]; TACHIKAWA KOUICHI [JP]; YUNOKI HIROYUKI [JP]; KOYAMA) 27 May 2004 (2004-05-27) * figure 2 * | 1,4,5 | |
| Y | EP 2 565 006 A2 (SUMITOMO RUBBER IND [JP]) 6 March 2013 (2013-03-06) | 3 | |
| A | * paragraph [0038]; figure 1 * | 1,2,4,5 | |

The present search report has been drawn up for all claims

| Place of search | Date of completion of the search | Examiner |
|---|---|---|
| Munich | 10 February 2016 | Diamantouros, S |

EPO FORM 1503 03.82 (P04C01)

## ANNEX TO THE EUROPEAN SEARCH REPORT
## ON EUROPEAN PATENT APPLICATION NO.

EP 15 18 8759

This annex lists the patent family members relating to the patent documents cited in the above-mentioned European search report.
The members are as contained in the European Patent Office EDP file on
The European Patent Office is in no way liable for these particulars which are merely given for the purpose of information.

10-02-2016

| Patent document cited in search report | | Publication date | Patent family member(s) | | Publication date |
|---|---|---|---|---|---|
| WO 2009128265 | A1 | 22-10-2009 | JP WO | 5459204 B2 2009128265 A1 | 02-04-2014 22-10-2009 |
| EP 2781231 | A2 | 24-09-2014 | EP JP US | 2781231 A2 2014180511 A 2014288508 A1 | 24-09-2014 29-09-2014 25-09-2014 |
| EP 2703025 | A1 | 05-03-2014 | CN EP JP US | 103656803 A 2703025 A1 2014047828 A 2014062036 A1 | 26-03-2014 05-03-2014 17-03-2014 06-03-2014 |
| EP 2703024 | A1 | 05-03-2014 | EP JP US WO | 2703024 A1 2012228335 A 2014066859 A1 2012147545 A1 | 05-03-2014 22-11-2012 06-03-2014 01-11-2012 |
| WO 2011125133 | A1 | 13-10-2011 | NONE | | |
| JP 2012147859 | A | 09-08-2012 | JP JP | 5828639 B2 2012147859 A | 09-12-2015 09-08-2012 |
| WO 2004044464 | A1 | 27-05-2004 | AU WO | 2003277671 A1 2004044464 A1 | 03-06-2004 27-05-2004 |
| EP 2565006 | A2 | 06-03-2013 | CN EP JP JP KR US US | 102962914 A 2565006 A2 5717593 B2 2013049236 A 20130024735 A 2013053786 A1 2014228774 A1 | 13-03-2013 06-03-2013 13-05-2015 14-03-2013 08-03-2013 28-02-2013 14-08-2014 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2013049236 A
- JP 2014014698 A

- JP 2014209223 A